# EUROPÄISCHE PATENTSCHRIFT

(11) **EP 1 756 132 B1**
(45) Veröffentlichungstag und Bekanntmachung des Hinweises auf die Patenterteilung: **19.12.2007**
(21) Anmeldenummer: 05740384.2
(22) Anmeldetag: 20.05.2005
(51) Int. Cl.: C07H 3/04, A61K 31/295

(54) **VERFAHREN ZUR HERSTELLUNG VON EISENSACCHAROSEKOMPLEX**
PROCESS FOR PREPARING AN IRON SACCHAROSE COMPLEX
PROCEDE DE PREPARATION D'UN COMPLEXE DE FER SACCHAROSE

(30) Priorität: 24.05.2004 WO PCT/CH2004/000313
(43) Veröffentlichungstag der Anmeldung: 28.02.2007
(73) Patentinhaber: Cilag AG, 8205 Schaffhausen (CH)
(72) Erfinder: JUSTUS, Michael, CH-8203 Schaffhausen (CH)
(74) Vertreter: Braun, André jr.
(86) Internationale Anmeldenummer: PCT/CH2005/000286
(87) Internationale Veröffentlichungsnummer: WO 2005/116040

(56) Entgegenhaltungen:
- WO-A-20/05000210
- DE-A1- 2 240 782
- DATABASE CHEMABS CHEMICAL ABSTRACTS SERVICE, COLUMBUS, OHIO, US; "An improved process for preparing saccharated iron oxide in powder form for use in tablets as well as for syrup preparation" XP002347464 gefunden im STN Database accession no. 141:416131 & IN 187 116 A (ALKEM LABS INDIA) 9. Februar 2002 (2002-02-09)

## Beschreibung

Die vorliegende Erfindung betrifft ein Verfahren zur Herstellung von Eisensaccharosekomplex, d.h. von Eisen(II)-saccharosekomplex und Eisen(III)saccharosekomplex, vorzugsweise von Eisen(III)saccharosekomplex. Eisen(III)-saccharosekomplex kann auch als Eisensaccharosekomplex, als Natrium-Eisensaccharose, bzw. als Natrium-Eisen(III)-saccharose, oder als Eisensucrose bzw. Eisen(III)-sucrose bezeichnet werden. Der in der Beschreibung gebrauchte Ausdruck Eisen(III)saccharosekomplex schliesst allgemein auch Eisen(II)saccharosekomplex mit ein.

Eisensaccharosekomplex, insbesondere Eisen(III)saccharosekomplex, sowie Verfahren zu dessen Herstellung sind an sich bekannt. Als Eisen(III)saccharosekomplex wird hierin das Umsetzungsprodukt von Eisenoxyhydroxid, insbesondere von Eisen(III)oxyhydroxid, mit Saccharose, insbesondere mit D(+)-Saccharose, bezeichnet. Als D(+)-Saccharose wird der Zucker D-Sucrose bzw. die Verbindung beta-D-Fructofuranosyl-alfa-D-glucopyranosid bezeichnet. Im weiteren wird der Ausdruck "Sucrose" oder "D-Sucrose" verwendet. Eisen(III)saccharosekomplex enthält organisch gebundenes Eisen und wird z.B. als Arzneimittel zur Erhöhung des Eisengehalts im Blut bei Menschen und Tieren eingesetzt.

In der offenlegerungschrift DE 38 44 065 A1 wird die Herstellung eines Eisensaccharosekomplexs aus Saccharose und Eisensalzen beschrieben

Die bekannten Verfahren haben bedeutende Nachteile. Als eines der Hauptprobleme erweist sich bei allen Verfahren zur Herstellung von Eisen(III)saccharosekomplex das Entfernen des aus dem eingesetzten Eisensalz (z.B. Eisenchlorid) resultierenden Anions (z.B. des Chloridgehalts), bzw. das Entfernen des entstandenen Gegenions, aus dem Eisenoxyhydroxid. Dieser Gehalt an Anionen ist physiologisch unerwünscht. In den bekannten Verfahren wird dieser Chloridgehalt jeweils sofort aus dem aufgeschlämmten Eisen(III)oxyhydroxid entfernt. Erfahrungsgemäss ist es jedoch sehr schwierig, frisch gefälltes kolloidales Eisenoxyhydroxid zu filtrieren. Gealtertes Eisenoxyhydroxid lässt sich zwar gut filtrieren, ist aber für die Synthese des physiologisch wirksamen Eisen(III)saccharosekomplexes ungeeignet. Darum wird das erhaltene Eisenoxyhydroxid mehrfach geschlämmt und die überstehende Lösung abdekantiert. Dieses Vorgehen ist technisch unpraktisch und aufwändig.

Mit dem vorliegenden erfindungsgemässen Verfahren wird dieser Nachteil behoben. Insbesondere ist es nicht nötig, den Chloridgehalt (oder andere Gegenionen bzw. Fremdsalze) aus dem frisch gefällten kolloidalen Eisenoxyhydroxid zu entfernen. Im weiteren erhält man den festen Eisen(III)-saccharosekomplex durch einfache Fällung, beispielsweise mit Hilfe eines organischen Lösungsmittels, so dass der erfindungsgemäss hergestellte Eisen(III)saccharosekomplex keine unerwünschten Träger- oder Zusatzstoffe enthält. Die Handhabung des Eisen(III)saccharosekomplexes als Feststoff ist wesentlich leichter und sicherer als in Form einer Lösung, da der Feststoff ohne Zersetzung über weite Strecken transportiert werden kann und wesentlich weniger Volumen beansprucht. Die Gefahr mikrobiologischer Verunreinigung besteht bei einem Feststoff im Gegensatz zu Lösungen kaum. Ebenso lässt sich ein Feststoff wesentlich besser aufreinigen und unerwünschte Nebenprodukte lassen sich besser entfernen als aus einer Lösung. Darum ist es vorteilhafter, das Produkt als Feststoff ohne Zusatz irgendwelcher Fremd- oder Hilfsstoffe zu fällen.

Gemäss dem erfindungsgemässen Verfahren ist es nicht erforderlich, die Base portionenweise bei bestimmten Temperaturen oder verschiedene Basen in bestimmter Reihenfolge zuzusetzen. Es genügt, das Reaktionsgemisch zur Bildung des Eisen(III)saccharosekomplexes zu erwärmen, vorzugsweise auf Rückflusstemperatur. Ein Abdampfen des Lösungsmittels, d.h. des Wassers, ist nicht erforderlich. Zur Fällung des Produktes ist es ausreichend, die Lösung zu einem mit Wasser mischbaren organischen Lösungsmittel, bevorzugt einem Alkohol zu geben oder ein mit Wasser mischbares organisches Lösungsmittel, bevorzugt einen Alkohol zur Lösung hinzu zu fügen. Dabei fällt der in der Lösung gebildete Eisen(III)saccharosekomplex unverändert aus. Je nach der Konzentration des Eisen(III)saccharosekomplexes ist dabei der Anteil an freier Sucrose im gefällten Produkt unterschiedlich. Bei Arbeiten in verdünnter Lösung wird die Lösung bevorzugt so weit aufkonzentriert, dass ein gut fliessfähiges Konzentrat zurückbleibt. Bei Arbeiten in konzentrierter Lösung muss nicht aufkonzentriert werden.

Das erfindungsgemässe Verfahren ist in den Patentansprüchen definiert. Insbesondere betrifft die vorliegende Erfindung ein Verfahren zur Herstellung von Eisensaccharosekomplex, insbesondere von Eisen(III)saccharosekomplex, welches dadurch gekennzeichnet ist, dass man
(a) ein Eisensalz in wässeriger Lösung, vorzugsweise ein Eisen(III)salz, vorzugsweise Eisen(III)chlorid-hexahydrat, gleichzeitig oder in beliebiger Reihenfolge mit Sucrose, vorzugsweise mit D-Sucrose, und einer anorganischen Base, vorzugsweise Alkalicarbonat und/oder Alkalihydrogencarbonat, vorzugsweise bei niedriger Temperatur, d.h. bei einer Temperatur im Bereich von -10°C bis 40°C, vorzugsweise bei etwa 5-25°C, mischt, so dass das Reaktionsgemisch einen Säurewert (pH) im Bereich von 3<pH<12, vorzugsweise im Bereich von 5<pH<9, und vorzugsweise etwa 7, aufweist, und das Reaktionsgemisch so lange bei diesem Säurewert belässt, bis alles Eisensalz in Eisenoxyhydroxid umgewandelt ist; wobei bei der Verwendung von Alkalihydroxid immer Sucrose zu Beginn der Reaktion vorgelegt oder gleichzeitig mit dem Alkalihydroxid dem Reaktionsgemisch zugesetzt wird;
(b) anschliessend den Säurewert (pH) des Reaktionsgemisches auf einen Wert im Bereich von 10<pH<12 erhöht, vorzugsweise durch Zusetzen von Alkalihydroxid und/oder Alkalicarbonat und/oder Alkalihydrogencarbonat und/oder Ammoniumhydroxid, vorzugsweise durch Zusetzen von Alkalihydroxid, insbesondere Natriumhydroxid, und das Reaktionsgemisch so lange erhitzt bis sich der gewünschte Eisensaccharosekomplex, vorzugsweise Eisen(III)saccharosekomplex, vollständig gebildet hat, vorzugsweise auf eine Temperatur im Bereich von 70°C bis Rückflusstemperatur oder unter Druck bis 140°C; und anschliessend durch Mischen mit einem geeigneten mit Wasser mischbaren Lösungsmittel, welches vorzugsweise eine Dielektrizitätskonstante im Bereich von 10-50 (bei 20°C) aufweist, oder mit einem Gemisch solcher Lösungsmittel, den gebildeten Eisensaccharosekomplex ausfällt, wobei der Eisensaccharosekomplex von den anwesenden Anionen sowie von einem allfälligen Überschuss der Base vor oder nach der Ausfällung in an sich bekannter Weise gereinigt wird.

Wird beispielsweise das Eisensalz in wässeriger Lösung gleichzeitig mit Sucrose vorgelegt und anschliessend mit der anorganischen Base umgesetzt, so wird davon ausgegangen, dass sich intermediär Eisenoxyhydroxid bildet. Die vorliegende Erfindung ist aber nicht an diese Erklärung gebunden.

Vorzugsweise geht man so vor, dass man
(al) ein Eisensalz, vorzugsweise ein Eisen(III)-salz, in wässeriger Lösung, vorzugsweise Eisen(III)chlorid-hexahydrat in wässeriger Lösung, mit einem Alkalicarbonat, vorzugsweise mit 1.5-5.0 Äquivalent, vorzugsweise mit 1.5-2.0 Äquivalent der Carbonatbase und/oder einem Alkalihydrogencarbonat, vorzugsweise mit 3.0-10.0 Äquivalent, vorzugsweise mit 3.0-4.0 Äquivalent einer Hydrogencarbonatbase, jeweils pro Äquivalent Eisenion, vorzugsweise bei niedriger Temperatur, mischt, so dass das Reaktionsgemisch einen Säurewert (pH) im Bereich von etwa 3<pH<12, vorzugsweise von 5<pH<9 aufweist, das Reaktionsgemisch so lange bei diesem Säurewert belässt, bis alles Eisensalz, vorzugsweise Eisen(III)chlorid-hexahydrat, in Eisenoxyhydroxid umgewandelt ist, gegebenenfalls das gebildete Eisenoxyhydroxid von den Fremdionen (z.B. Chloridionen) vorreinigt, beispielsweise mittels Dekantieren, Ionenaustausch, Filtration oder Ultrafiltration, und anschliessend mit der notwendigen Menge Sucrose, vorzugsweise mit mindestens 2 Äquivalent, vorzugsweise mit 2.0-5.0 Äquivalent Sucrose, pro Äquivalent Eisenion, versetzt, vorzugsweise mit D-Sucrose;
(b1) anschliessend den Säurewert (pH) des Reaktionsgemisches auf eine Wert im Bereich von 10<pH<12 erhöht, vorzugsweise durch Zusetzen von Alkalihydroxid und/oder Alkalicarbonat und/oder Alkalihydrogencarbonat und/oder Ammoniumhydroxid, vorzugsweise von Natriumhydroxid, und das Reaktionsgemisch so lange erhitzt, bis sich der gewünschte Eisensaccharosekomplex, vorzugsweise Eisen(III)-saccharosekomplex, vollständig gebildet hat, vorzugsweise auf eine Temperatur im Bereich von 70°C bis Rückflusstemperatur, gegebenenfalls unter Druck bis auf eine Temperatur von 140°C, und anschliessend durch Mischen mit einem geeigneten mit Wasser mischbaren organischen Lösungsmittel, welches vorzugsweise eine Dielektrizitätskonstante im Bereich von 10-50 (bei 20°C) aufweist, oder mit einem Gemisch solcher Lösungsmittel, den gebildeten Eisensaccharosekomplex ausfällt, wobei der Eisensaccharosekomplex von gegebenenfalls noch anwesenden Chloridionen vor oder nach der Ausfällung in an sich bekannter Weise gereinigt wird.

Durch Mischen mit einem geeigneten mit Wasser mischbaren organischen Lösungsmittel bedeutet, dass man die Lösung zu einem mit Wasser mischbaren organischen Lösungsmittel, welches vorzugsweise eine Dielektrizitätskonstante im Bereich von 10-50 (bei 20°C) aufweist, oder einem Gemisch solcher Lösungsmittel, zugibt oder ein mit Wasser mischbares organisches Lösungsmittel, welches vorzugsweise eine Dielektrizitätskonstante im Bereich von 10-50 (bei 20°C) aufweist, oder ein Gemisch solcher Lösungsmittel, zur Lösung hinzufügt.

Der Begriff "ein mit Wasser mischbares organisches Lösungsmittel" im Sinne der vorliegenden Erfindung bedeutet ein mit Wasser mischbares organisches Lösungsmittel, oder ein Gemisch unterschiedlicher organischer Lösungsmittel, welches eine Dielektrizitätskonstante im Bereich von 10-50 (bei 20°C), vorzugsweise im Bereich von 20-50 (bei 20°C) aufweist. Vorzugsweise stellt diese Verbindung einen Alkohol oder ein Keton dar. Als Alkohol ist die Verbindung vorzugsweise ein primärer, sekundärer oder tertiärer C₍₁₋₆₎-Alkohol oder Benzylalkohol oder Ethylenglykol oder Propylenglykol oder Glycerin, vorzugsweise Methanol, Ethanol, n-Propanol, Isopropanol, n-Butanol, sec.-Butanol und/oder tert.-Butanol, vorzugsweise Methanol und Ethanol. Als Keton entspricht die Verbindung vorzugsweise der Formel C₍₁₋₃₎-Alkyl-C (O)-C₍₁₋₃₎-Alkyl, vorzugsweise der Formel C_{(1,2)}-Alkyl-C(O)-C_{(1,2)}-Alkyl und ist vorzugsweise Methylethylketon oder Aceton, vorzugsweise Aceton. Als "Gemisch solcher Lösungsmittel" sind vorzugsweise Gemische der vorgenannten Lösungsmittel zu verstehen, wobei ein vorgenanntes Lösungsmittel oder ein Gemisch solcher Lösungsmittel bevorzugt ist, welches etwa 20 Gew.-%, 40 Gew.-%, oder 60 Gew.-% Methanol oder Ethanol oder etwa 20 Gew.-%, 40 Gew.-%, oder 60 Gew.-% eines Gemisches von Methanol und Ethanol, enthält.

Die vorliegende Erfindung betrifft auch den derart hergestellten Eisensaccharosekomplex sowie dessen Verwendung zur Herstellung von Arzneimitteln, vorzugsweise für die Heilung anaemischer Zustände. Die vorliegende Erfindung betrifft auch Arzneimittel, welche erfindungsgemäss hergestellten Eisensaccharosekomplex enthalten.

Erfindungsgemäss kann das im leicht sauren bis mässig alkalischem wässerigem Medium gebildete Eisenoxyhydroxid *in situ,* oder nach vorgängiger Isolierung, jedoch ohne spezielle Reinigung von Anionen, z.B. von Chloridionen, direkt mit Sucrose zu Eisensaccharosekomplex, vorzugsweise zu Eisen(III)saccharosekomplex, umgesetzt werden. In der besonders bevorzugten Ausführungsform wird das im leicht sauren bis mässig alkalischem wässerigem Medium gebildete Eisenoxyhydroxid nicht isoliert, sondern *in situ* direkt mit Sucrose zum Eisensaccharosekomplex umgesetzt. Dabei wird der Eisensaccharosekomplex in kolloidaler Lösung erhalten. Diese Lösung kann, gegebenenfalls nach Aufkonzentrierung, von Fremdanteilen durch Filtration, Ultrafiltration, durch Ionenaustausch, Dialyse oder durch eine andere an sich bekannte Filtrationstechnik, von unerwünschten Bestandteilen, wie Anionen oder überschüssiger Base, vorgereinigt werden. Anstelle des Eisen(II)chlorid-hexahydrats oder des Eisen(III)chlorid-hexahydrats als Ausgangsmaterial können auch andere entsprechende Eisensalze, vorzugsweise Eisen(III)salze, verwendet werden, jedoch hat sich die Verwendung von Eisen(III)chlorid-hexahydrat als vorteilhaft erwiesen, da diese Verbindung kostengünstig und einfach zu handhaben ist. Beispiele solcher in Wasser löslichen Eisensalze sind Eisennitrate, wie Eisen(III)-nitrat-hexahydrat oder Eisen(III)nitrat-nonahydrat.

Der nach der Fällung erhaltene Eisen(III)saccharosekomplex kann in einfacher Weise weiter gänzlich frei von Fremdstoffen (z.B. chloridfrei) gereinigt und/oder gewaschen werden. Überraschend ist aber, das die Fällung bereits ein sehr reines Produkt ergibt, welches den Spezifikationen genügt und eine weitere Reinigung in der Regel nicht nötig ist. Entscheidend ist, dass erfindungsgemäss erst der fertige Eisen(III)saccharosekomplex von anwesenden Chloridionen befreit werden muss, was erheblich einfacher ist, als die Umfällung oder Schlämmung des feuchten Eisenoxyhydroxids.
Der im Verfahren erhaltene Niederschlag an gefälltem Eisenoxyhydroxid kann isoliert oder direkt weiter verarbeitet werden. Zum wässerigen Eisenoxyhydroxid werden mindestens zwei Äquivalente Sucrose (pro Äquivalent Eisenion) sowie Natronlauge bis zur deutlich basischen Reaktion, bevorzugt bis zu einem pH-Wert von 10.0-12.0 hinzu gesetzt und der Ansatz auf mindestens 70°C, vorzugsweise auf Rückfluss erhitzt. Es kann gegebenenfalls auch bei erhöhtem Druck bis auf 140°C erwärmt werden. Um die gewünschten Eigenschaften des Produktes zu erhalten (Molekulargewicht, pH-Wert, Farbe, Säurestabilität) wird der Ansatz für mindestens 0.2 Stunden, vorzugsweise für 0.5-96 Stunden, vorzugsweise für etwa 1-8 Stunden, vorzugsweise für etwa 6-8 Stunden, auf Rückflusstemperatur erwärmt. Dabei bildet sich der gewünschte Komplex, bzw. der gewünschte Eisen(III)saccharosekomplex, welcher gemäss Analytik für die parenterale Eisentherapie geeignet ist. Gegebenenfalls wird dann der gebildete Eisen(III)saccharosekomplex mit üblichen Methoden, wie z.B. Filtration, Ultrafiltration, Ionenaustausch oder Dialyse, oder einer andern bekannten Methode, von Fremdsalzen gereinigt. Anschliessend wird der Ansatz gegebenenfalls aufkonzentriert, so dass dieser gut fliessfähig bleibt. Nach dem Entfernen der Fremdsalze kann die wässrige Lösung des erhaltenen Komplexes, gegebenenfalls vor der Fällung und/oder dem Aufkonzentrieren, nochmals für eine für die Erreichung des Zweckes notwendige Zeitdauer, vorzugsweise auf eine Temperatur im Bereich von 70°C bis Rückflusstemperatur, gegebenenfalls unter Druck bis auf eine Temperatur von 140°C erhitzt werden. Diese Erhitzung kann beispielsweise durchgeführt werden, um das Produkt einer Dampfsterilisation zu unterziehen, oder die Lösung einzuengen bzw. aufzukonzentrieren, oder um eine bessere Fliessfähigkeit der Lösung zu erhalten, oder um das Molekulargewicht des Produktes zu optimieren. Durch Zugabe eines mit Wasser mischbaren Lösungsmittels, oder eines Gemisches solcher Lösungsmittel, wie dieses vorgehend definiert ist, kann der Wirkstoff aber auch direkt gefällt und abfiltriert oder abzentrifugiert und gegebenenfalls gereinigt werden. Während des Aufkonzentrierens und der Fällung ändert sich der Komplex nicht mehr.

Die folgenden Beispiele zur Herstellung der erfindungsgemässen Eisen(III)saccharosekomplexes erläutern die Erfindung.

### Beispiel 1

22.61 g (83.6 mmol) Eisenchlorid-hexahydrat werden bei Raumtemperatur in 80 ml Wasser gelöst. Eine Lösung von 13.3 g (125.4 mmol) Natriumcarbonat in 78.4 g Wasser wird in einem Temperaturbereich von -5°C bis 25°C hinzugefügt, so dass die Lösung nicht zu stark schäumt. Nach Zugabe von etwa einem Drittel der Lösung beginnt ein flockiger Niederschlag auszufallen. Nach vollständiger Zugabe der Base wird die entstandene Suspension so lange nachgerührt, bis die Gasentwicklung aufhört. Dann gibt man 85.86 g (250.8 mmol) D-Sucrose und 27.03 g (202.7 mmol) 30%ige wässrige Natronlauge zum Ansatz und erhitzt diesen für drei Stunden auf Rückflusstemperatur. Der Ansatz wird auf 60% seines ursprünglichen Volumens aufkonzentriert und durch Zugabe auf 860 g Methanol unter langsamen Abkühlen bei 20-40°C ausgefällt. Die entstandene braune Suspension wird über Nacht bei Raumtemperatur gerührt, abfiltriert und mit Methanol nachgewaschen. Das Produkt wird bei 50°C im Vakuum getrocknet. Man erhält 45.52 g eines braunen Pulvers, dass gemäss Analytik (u.a. Gelpermeationschromatographie) für die parenterale Eisentherapie geeignet ist. Sollte der Chloridgehalt des Produktes ausserhalb der Spezifikation liegen, wird das Produkt einmal in einer Mischung aus Methanol:Wasser 6:1 nachgeschlämmt.

### Beispiel 2

22.61 g (83.6 mmol) Eisenchlorid-hexahydrat werden bei Raumtemperatur in 80 ml Wasser gelöst. Eine Lösung von 13.3 g (125.4 mmol) Natriumcarbonat in 78.4 ml Wasser wird in einem Temperaturbereich von -5°C bis 25°C hinzugefügt, so dass die Lösung nicht zu stark schäumt. Nach Zugabe von etwa einem Drittel der Lösung beginnt ein flockiger Niederschlag auszufallen. Nach vollständiger Zugabe der Base wird die entstandene Suspension so lange nachgerührt, bis die Gasentwicklung aufhört. Dann gibt man 85.86 g (250.8 mmol) D-Sucrose und 27.40 g (205.5 mmol) 30%ige wässrige Natronlauge zum Ansatz und erhitzt den Ansatz für zwei Stunden unter Druck auf 120-130°C. Der Ansatz wird auf 60% seines ursprünglichen Volumens aufkonzentriert und durch Zugabe von 573 g Methanol unter langsamen Abkühlen bei 20-40°C ausgefällt. Die entstandene braune Suspension wird über Nacht bei Raumtemperatur gerührt, abfiltriert und mit Methanol nachgewaschen. Das Produkt wird bei 50°C im Vakuum getrocknet. Man erhält 45.85 g eines braunen Pulvers, dass gemäss Analytik (u. a. Gelpermeationschromatographie) für die parenterale Eisentherapie geeignet ist. Sollte der Chloridgehalt des Produktes ausserhalb der Spezifikation liegen, wird das Produkt einmal in einer Mischung aus Methanol:Wasser 6:1 nachgeschlämmt.

### Beispiel 3

22.61 g (83.6 mmol) Eisenchlorid-hexahydrat werden bei Raumtemperatur in 80 ml Wasser gelöst. Eine Lösung von 13.3 g (125.4 mmol) Natriumcarbonat in 78.4 ml Wasser wird in einem Temperaturbereich von -5°C bis 25°C hinzugefügt, so dass die Lösung nicht zu stark schäumt. Nach Zugabe von etwa einem Drittel der Lösung beginnt ein flockiger Niederschlag auszufallen. Nach vollständiger Zugabe der Base wird die entstandene Suspension so lange nachgerührt, bis die Gasentwicklung aufhört. Dann gibt man 85.86 g (250.8 mmol) D-Sucrose und 19.87 g (149 mmol) 30%ige wässrige Natronlauge zum Ansatz und erhitzt den Ansatz für 30 Minuten auf Rückflusstemperatur. Der Ansatz wird in Dialyseschläuche gefüllt und über Nacht in leichtem Wasserstrom dialysiert. Dann wird auf 35% des ursprünglichen Volumens aufkonzentriert und nochmals für 6 Stunden unter Rückfluss erhitzt. Durch Zugabe von 396 g Methanol unter langsamen Abkühlen bei 20-40°C ausgefällt. Die entstandene braune Suspension wird über Nacht bei Raumtemperatur gerührt, abfiltriert und mit Methanol nachgewaschen. Das Produkt wird bei 50°C im Vakuum getrocknet. Man erhält 65.80 g eines braunen Pulvers, dass gemäss Analytik (u.a. Gelpermeationschromatographie) für die parenterale Eisentherapie geeignet ist.

### Beispiel 4

19.79 g (73.2 mmol) Eisenchlorid-hexahydrat werden bei Raumtemperatur in 100 ml Wasser gelöst. Eine Lösung von 11.43 g (107.8 mmol) Natriumcarbonat in 100 ml Wasser wird in einem Temperaturbereich von -5°C bis 25°C hinzugefügt, so dass die Lösung nicht zu stark schäumt. Nach Zugabe von etwa zwei Drittel der Lösung beginnt ein flockiger Niederschlag auszufallen. Nach vollständiger Zugabe der Base wird die entstandene Suspension so lange nachgerührt, bis die Gasentwicklung aufhört. Dann gibt man 2.8 g (21 mmol) 30%ige Natronlauge hinzu, filtriert den ausgefallenen Feststoff ab und wäscht mit Wasser nach (Feststoff braucht nicht chloridfrei gewaschen zu werden). Das braune, noch stark wasserhaltige Feuchtprodukt wird in 90 ml Wasser aufgeschlämmt, 60.12 g (175.6 mmol) D-Sucrose und 7.3 g (54.8 mmol) 30%ige wässrige Natronlauge zum Ansatz und erhitzt den Ansatz für 6 Stunden auf Rückflusstemperatur. Der Ansatz wird auf 40% seines ursprünglichen Volumens aufkonzentriert und durch Zugabe auf 500 g Methanol unter langsamen Abkühlen bei 20-40°C ausgefällt. Die entstandene braune Suspension wird über Nacht bei Raumtemperatur gerührt, abfiltriert und mit Methanol nachgewaschen. Das Produkt wird bei 50°C im Vakuum getrocknet. Man erhält 48.5 g eines braunen Pulvers, dass gemäss Analytik (u.a. Gelpermeationschromatographie) für die parenterale Eisentherapie geeignet ist.

### Beispiel 5

19.79 g (73.2 mmol) Eisenchlorid-hexahydrat werden bei Raumtemperatur in 100 ml Wasser gelöst. Eine Lösung von 11.5 g (108.3 mmol) Natriumcarbonat in 100 ml Wasser wird in einem Temperaturbereich von -5°C bis 25°C hinzugefügt, so dass die Lösung nicht zu stark schäumt. Nach Zugabe von etwa zwei Drittel der Lösung beginnt ein flockiger Niederschlag auszufallen. Nach vollständiger Zugabe der Base wird die entstandene Suspension so lange nachgerührt, bis die Gasentwicklung aufhört. Der ausgefallene Feststoff wird abfiltriert und mit Wasser nachgewaschen (Feststoff braucht nicht chloridfrei gewaschen zu werden). Das braune, noch stark wasserhaltige Feuchtprodukt wird in 80 ml Wasser aufgeschlämmt, 60.48 g (176.8 mmol) D-Sucrose und 5.69 g (42.7 mmol) 30%ige wässrige Natronlauge werden zum Ansatz hinzugegeben und man erhitzt den Ansatz für 3 Stunden auf Rückflusstemperatur. Der Ansatz wird auf 40% seines ursprünglichen Volumens aufkonzentriert und durch Zugabe auf 113.3 g Ethanol unter langsamen Abkühlen bei 20-40°C ausgefällt. Die entstandene braune Suspension wird 1.5 Stunden bei Raumtemperatur gerührt, abfiltriert und mit Ethanol nachgewaschen. Das Produkt wird bei 50°C im Vakuum getrocknet. Man erhält 60.09 g eines braunen Pulvers, dass gemäss Analytik (u.a. Gelpermeationschromatographie) für die parenterale Eisentherapie geeignet ist.

### Beispiel 6

178.11 g (659 mmol) Eisenchlorid-hexahydrat werden bei Raumtemperatur in 900 ml Wasser gelöst. Eine Lösung von 166.2 g (1976 mmol) Natriumhydrogencarbonat in 2050 ml Wasser wird in einem Temperaturbereich von -5°C bis 25°C hinzugefügt, so dass die Lösung nicht zu stark schäumt. Gegen Ende der Zugabe der Lösung beginnt ein flockiger Niederschlag auszufallen. Nach vollständiger Zugabe der Base wird die entstandene Suspension so lange nachgerührt, bis die Gasentwicklung aufhört. Der ausgefallene Feststoff wird abfiltriert und mit Wasser nachgewaschen (Feststoff braucht nicht chloridfrei gewaschen zu werden). Das braune, noch stark wasserhaltige Feuchtprodukt wird in 635 ml Wasser aufgeschlämmt, 540 g (1579 mmol) D-Sucrose werden hinzugegeben, der pH-Wert durch Zugabe von 30%iger Natronlauge auf 11.5-12.0 eingestellt. Anschliessend wird der Ansatz für 8 Stunden auf Rückflusstemperatur erhitzt. Der Ansatz wird auf 40% seines ursprünglichen Volumens aufkonzentriert und durch Zugabe auf 4350 g Methanol unter langsamen Abkühlen bei 20-40°C ausgefällt. Die entstandene braune Suspension wird 2 Stunden bei Raumtemperatur gerührt, abfiltriert und mit Methanol nachgewaschen. Das Produkt wird bei 50°C im Vakuum getrocknet. Man erhält 476 g eines braunen Pulvers, dass gemäss Analytik (u.a. Gelpermeationschromatographie) für die parenterale Eisentherapie geeignet ist.

### Beispiel 7

Die vorgehenden Beispiele 1 bis 6 führt man durch, indem man für die Fällung als organisches Lösungsmittel n-Propanol, Isopropanol, n-Butanol, sec.-Butanol, tert.-Butanol, Benzylalkohol, Ethylenglykol, Propylenglykol, Glycerin, Aceton, oder ein Gemisch dieser Verbindungen, wie n-Propanol, Isopropanol, n-Butanol, sec.-Butanol, tert.-Butanol, Benzylalkohol, Ethylenglykol, Propylenglykol, Glycerin, Aceton, enthaltend 20 Gew.-%, 40 Gew.-%, oder 60 Gew.-% Methanol und/oder Ethanol, einsetzt.

## Patentansprüche

1. Verfahren zur Herstellung von Eisensaccharosekomplex, **dadurch gekennzeichnet ist, dass** man
(a) ein Eisensalz in wässeriger Lösung, gleichzeitig oder in beliebiger Reihenfolge mit Sucrose und einer anorganischen Base bei einer Temperatur im Bereich von -10°C bis 40°C mischt, so dass das Reaktionsgemisch einen Säurewert (pH) im Bereich von 3<pH<12 aufweist, und das Reaktionsgemisch so lange bei diesem Säurewert belässt, bis alles Eisensalz in Eisenoxyhydroxid umgewandelt ist; wobei bei der Verwendung von Alkalihydroxid immer Sucrose zu Beginn der Reaktion vorgelegt oder gleichzeitig mit dem Alkalihydroxid dem Reaktionsgemisch zugesetzt wird;
(b) anschliessend den Säurewert (pH) des Reaktionsgemisches auf eine Wert im Bereich von 10<pH<12 erhöht, vorzugsweise durch Zusetzen von Alkalihydroxid und/oder Alkalicarbonat und/oder Alkalihydrogencarbonat und/oder Ammoniumhydroxid, und das Reaktionsgemisch so lange erhitzt bis sich der gewünschte Eisensaccharosekomplex vollständig gebildet hat; und anschliessend durch Mischen mit einem geeigneten mit Wasser mischbaren Lösungsmittel den gebildeten Eisensaccharosekomplex ausfällt, wobei der Eisensaccharosekomplex von den anwesenden Anionen sowie von einem allfälligen Überschuss der Base vor oder nach der Ausfällung gereinigt wird.

2. Verfahren nach Anspruch 1, **dadurch gekennzeichnet, dass** man Eisen(III)saccharosekomplex herstellt.

3. Verfahren nach Anspruch 1 oder 2, **dadurch gekennzeichnet, dass** man in der Stufe (a) ein Eisen(III)salz, vorzugsweise Eisen (III) chlorid-hexahydrat, mit D-Sucrose, und einer anorganischen Base, vorzugsweise Alkalicarbonat und/oder Alkalihydrogencarbonat, bei einer Temperatur im Bereich von 5-25°C, mischt, so dass das Reaktionsgemisch einen Säurewert im Bereich von 5<pH<9, und vorzugsweise etwa 7, aufweist, und das Reaktionsgemisch so lange bei diesem Säurewert belässt, bis alles Eisensalz in Eisenoxyhydroxid umgewandelt ist.

4. Verfahren nach einem der Ansprüche 1-3, **dadurch gekennzeichnet, dass** man in Stufe (b) den Säurewert des Reaktionsgemisches auf eine Wert im Bereich von 10<pH<12 durch Zusetzen von Alkalihydroxid, vorzugsweise Natriumhydroxid, erhöht und das Reaktionsgemisch auf eine Temperatur im Bereich von 70°C bis Rückflusstemperatur oder unter Druck bis 140°C erhitzt.

5. Verfahren nach einem der Ansprüche 1-4, **dadurch gekennzeichnet, dass** man in Stufe (b) für die Fällung des Eisensaccharosekomplexes ein mit Wasser mischbares organisches Lösungsmittel oder ein Gemisch solcher Lösungsmittel verwendet, welches eine Dielektrizitätskonstante im Bereich von 20-50 (bei 20°C) aufweist.

6. Verfahren nach Anspruch 5, **dadurch gekennzeichnet, dass** dieses Lösungsmittel ein Alkohol ist, vorzugsweise ein primärer, sekundärer oder tertiärer C₍₁₋₆₎-Alkohol oder Benzylalkohol oder Ethylenglykol oder Propylenglykol oder Glycerin, vorzugsweise Methanol, Ethanol, n-Propanol, Isopropanol, n-Butanol, sec.-Butanol und/oder tert.-Butanol, vorzugsweise Methanol und Ethanol.

7. Verfahren nach Anspruch 5, **dadurch gekennzeichnet, dass** dieses Lösungsmittel ein Keton ist, vorzugsweise eine Verbindung der Formel C₍₁₋₃₎-Alkyl-C(O)-C₍₁₋₃₎-Alkyl, vorzugsweise eine Verbindung der Formel C_{(1,2)}-Alkyl-C(O)-C_{(1,2)}-Alkyl, vorzugsweise Methylethylketon oder Aceton, vorzugsweise Aceton.

8. Verfahren nach Anspruch 6 oder 7, **dadurch gekennzeichnet, dass** ein Gemisch dieser Lösungsmittel eingesetzt wird, vorzugsweise ein einzelnes Lösungsmittel oder ein Gemisch dieser Lösungsmittel, welches etwa 20 Gew.-%, 40 Gew.-%, oder 60 Gew.-% Methanol oder Ethanol oder etwa 20 Gew.-%, 40 Gew.-%, oder 60 Gew.-% eines Gemisches von Methanol und Ethanol, enthält.

9. Verfahren nach einem der Ansprüche 1-8, **dadurch gekennzeichnet, dass** man in Stufe (a) das Eisensalz mit einem Alkalicarbonat, vorzugsweise mit 1.5-5.0 Äquivalent, vorzugsweise mit 1.5-2.0 Äquivalent der Carbonatbase und/oder einem Alkalihydrogencarbonat, vorzugsweise mit 3.0-10.0 Äquivalent, vorzugsweise mit 3.0-4.0 Äquivalent einer Hydrogencarbonatbase, jeweils pro Äquivalent Eisenion, mischt, das Reaktionsgemisch so lange beim eingestellten Säurewert belässt, bis alles Eisensalz in Eisenoxyhydroxid umgewandelt ist, gegebenenfalls das gebildete Eisenoxyhydroxid von den Fremdionen vorreinigt und anschliessend mit mindestens 2 Äquivalent, vorzugsweise mit 2.0-5.0 Äquivalent Sucrose, pro Äquivalent Eisenion, versetzt.

10. Verfahren nach Anspruch 9, **dadurch gekennzeichnet, dass** man das gebildete Eisenoxyhydroxid von den Fremdionen mittels Dekantieren, Ionenaustausch, Filtration oder Ultrafiltration, vorreinigt,

11. Verfahren nach einem der Ansprüche 1-10, **dadurch gekennzeichnet, dass** das gebildete Eisenoxyhydroxid *in situ,* oder nach vorgängiger Isolierung, jedoch ohne spezielle Reinigung von Anionen, direkt mit Sucrose zu Eisensaccharosekomplex umgesetzt wird.

12. Verfahren nach Anspruch 11, **dadurch gekennzeichnet dass** das gebildete Eisenoxyhydroxid *in situ* direkt mit Sucrose zu Eisensaccharosekomplex umgesetzt wird.

13. Verfahren nach einem der Ansprüche 1-12, **dadurch gekennzeichnet, dass** man in Stufe (b) den Ansatz für mindestens 0.2 Stunden, vorzugsweise für 0.5-96 Stunden, vorzugsweise für etwa 1-8 Stunden, vorzugsweise für etwa 6-8 Stunden, auf Rückflusstemperatur erwärmt.

14. Verfahren nach einem der Ansprüche 1-13, **dadurch gekennzeichnet, dass** die kolloidale Lösung enthaltend den Eisensaccharosekomplex von Fremdanteilen durch Filtration, Ultrafiltration, durch Ionenaustausch, Dialyse oder durch eine andere an sich bekannte Filtrationstechnik, reinigt.

15. Verfahren nach einem der Ansprüche 1-14, **dadurch gekennzeichnet, dass** man die wässrige Lösung des erhaltenen Komplexes nach dem Entfernen der Fremdsalze auf eine Temperatur im Bereich von 70°C bis Rückflusstemperatur, gegebenenfalls unter Druck bis auf eine Temperatur von 140°C erhitzt.

16. Verfahren nach einem der Ansprüche 1-15, **dadurch gekennzeichnet, dass** man den erhaltenen Komplex direkt fällt, abfiltriert oder abzentrifugiert und anschliessend reinigt.

## Claims

1. Process for preparing iron-sucrose complex, **characterized in that**
(a) an iron salt is mixed in aqueous solution, simultaneously or in any sequence, with sucrose and an inorganic base at a temperature in the range from -10°C to 40°C, so that the reaction mixture has an acid value (pH) in the range 3<pH<12, and the reaction mixture is left at this acid value until all the iron salt is converted into iron oxyhydroxide; where on use of alkali metal hydroxide sucrose is always present at the start of the reaction or is added to the reaction mixture at the same time as the alkali metal hydroxide;
(b) subsequently the acid value (pH) of the reaction mixture is raised to a value in the range 10<pH<12, preferably by adding alkali metal hydroxide and/or alkali metal carbonate and/or alkali metal bicarbonate and/or ammonium hydroxide, and the reaction mixture is heated until the desired iron-sucrose complex has completely formed; and subsequently the iron-sucrose complex formed is precipitated by mixing with a suitable water-miscible solvent, where the iron-sucrose complex is purified from the anions present and from any excess of base before or after the precipitation.

2. Process according to Claim 1, **characterized in that** iron(III)-sucrose complex is prepared.

3. Process according to Claim 1 or 2, **characterized in that** in stage (a) an iron(III) salt, preferably iron(III) chloride hexahydrate, is mixed with D-sucrose, and with an inorganic base, preferably alkali metal carbonate and/or alkali metal bicarbonate, at a temperature in the range 5-25°C, so that the reaction mixture has an acid value in the range 5<pH<9, and preferably about 7, and the reaction mixture is left at this acid value until all the iron salt is converted into iron oxyhydroxide.

4. Process according to any of Claims 1-3, **characterized in that** in stage (b) the acid value of the reaction mixture is raised to a value in the range 10<pH<12 by adding alkali metal hydroxide, preferably sodium hydroxide, and the reaction mixture is heated to a temperature in the range from 70°C to the reflux temperature or under pressure to 140°C.

5. Process according to any of Claims 1-4, **characterized in that** in stage (b) a water-miscible organic solvent or a mixture of such solvents which has a dielectric constant in the range 20-50 (at 20°C) is used for precipitating the iron-sucrose complex.

6. Process according to Claim 5, **characterized in that** this solvent is an alcohol, preferably a primary, secondary or tertiary C₍₁₋₆₎ alcohol or benzyl alcohol or ethylene glycol or propylene glycol or glycerol, preferably methanol, ethanol, n-propanol, isopropanol, n-butanol, sec-butanol and/or tert-butanol, preferably methanol and ethanol.

7. Process according to Claim 5, **characterized in that** this solvent is a ketone, preferably a compound of the formula C₍₁₋₃₎-alkyl-C(O)-C₍₁₋₃₎-alkyl, preferably a compound of the formula C₍₁₋₂₎-alkyl-C(O)-C_{(1,2)}-alkyl, preferably methyl ethyl ketone or acetone, preferably acetone.

8. Process according to Claim 6 or 7, **characterized in that** a mixture of these solvents is employed, preferably a single solvent or a mixture of these solvents which comprises about 20% by weight, 40% by weight, or 60% by weight of methanol or ethanol or about 20% by weight, 40% by weight, or 60% by weight of a mixture of methanol and ethanol.

9. Process according to any of Claims 1-8, **characterized in that** in stage (a) the iron salt is mixed with an alkali metal carbonate, preferably with 1.5-5.0 equivalent, preferably with 1.5-2.0 equivalent, of the carbonate base and/or with an alkali metal bicarbonate, preferably with 3.0-10.0 equivalent, preferably with 3.0-4.0 equivalent, of a bicarbonate base, in each case per equivalent of iron ion, the reaction mixture is left at the adjusted acid value until all the iron salt is converted into iron oxyhydroxide, where appropriate the iron oxyhydroxide formed is prepurified from the foreign ions, and subsequently at least 2 equivalent, preferably 2.0-5.0 equivalent, of sucrose per equivalent of iron ion are added.

10. Process according to Claim 9, **characterized in that** the iron oxyhydroxide formed is prepurified from the foreign ions by decantation, ion exchange, filtration or ultra filtration.

11. Process according to any of Claims 1-10, **characterized in that** the iron oxyhydroxide formed is reacted *in situ* or after previous isolation, but without specific purification from anions, directly with sucrose to give iron-sucrose complex.

12. Process according to Claim 11, **characterized in that** the iron oxyhydroxide formed is reacted *in situ* directly with sucrose to give iron-sucrose complex.

13. Process according to any of Claims 1-12, **characterized in that** in stage (b) the mixture is heated at the reflux temperature for at least 0.2 hours, preferably for 0.5-96 hours, preferably for about 1-8 hours, preferably for about 6-8 hours.

14. Process according to any of Claims 1-13, **characterized in that** the colloidal solution comprising the iron-sucrose complex is purified from foreign fractions by filtration, ultra filtration, by ion exchange, dialysis or by another filtration technique known per se.

15. Process according to any of Claims 1-14, **characterized in that** the aqueous solution of the resulting complex is, after removal of the foreign salts, heated at a temperature in the range from 70°C to the reflux temperature, where appropriate under pressure up to a temperature of 140°C.

16. Process according to any of Claims 1-15, **characterized in that** the resulting complex is directly precipitated, removed by filtration or centrifugation and then purified.

## Revendications

1. Procédé pour la préparation d'un complexe de fer saccharose, **caractérisé en ce que**
(a) un sel de fer dans une solution aqueuse est mélangé, en même temps ou dans un ordre quelconque, avec du sucrose et une base inorganique à une température de l'ordre de -10°C à 40°C de telle façon que le mélange de réaction ait une valeur d'acidité (pH) de l'ordre de 3<pH<12 et que le mélange de réaction soit tenu à cette valeur d'acidité jusqu'à ce que tout le sel de fer soit transformé en oxyhydroxyde de fer ; où lors de l'utilisation d'alcali hydroxyde, du sucrose est toujours fourni au début de la réaction ou ajouté au mélange de réaction en même temps que l'alcali hydroxyde ;
(b) ensuite la valeur d'acidité (pH) du mélange de réaction est augmentée à une valeur de l'ordre de 10<pH<12, de préférence par l'addition d'alcali hydroxyde et/ ou d'alcali carbonate et/ ou d'alcali hydrogénocarbonate et/ ou d'hydroxyde d'ammonium, et le mélange de réaction est chauffé jusqu'à ce que le complexe de fer saccharose souhaité soit complètement formé ; et ensuite le complexe de fer saccharose formé est précipité par le mélange avec un solvant approprié qui peut être mélangé avec de l'eau, où le complexe de fer saccharose est nettoyé des anions présents ainsi que d'un éventuel reste de base avant ou après la précipitation.

2. Procédé selon la revendication 1, **caractérisé en ce que** l'on prépare un complexe de fer (III) saccharose.

3. Procédé selon les revendications 1 ou 2, **caractérisé en ce que** dans l'étape (a), un sel de fer (III), de préférence de l'hexahydrate de chlorure de fer (III) est mélangé avec du sucrose D et une base inorganique, de préférence du alcali carbonate et/ ou de l'alcali hydrogénocarbonate, à une température de l'ordre de 5 à 25°C de manière à ce que le mélange de réaction ait une valeur d'acidité de l'ordre de 5<pH<9, et de préférence environ 7, et que le mélange de réaction est tenu à cette valeur d'acidité jusqu'à ce que tout le sel de fer soit transformé en oxyhydroxyde de fer.

4. Procédé selon l'une quelconque des revendications 1 à 3, **caractérisé en ce que** dans l'étape (b) la valeur d'acidité du mélange de réaction est augmentée à une valeur de l'ordre de 10<pH<12 par l'addition d'alcali hydroxyde, de préférence d'hydroxyde de sodium, et que le mélange de réaction est chauffé à une température de l'ordre de 70°C jusqu'à la température de reflux ou sous pression jusqu'à 140°C.

5. Procédé selon l'une quelconque des revendications 1 à 4, **caractérisé en ce que** dans l'étape (b) est utilisé un solvant organique qui peut être mélangé avec de l'eau ou un mélange de tels solvants qui a une constante diélectrique de l'ordre de 20 à 50 (à 20°C) pour la précipitation du complexe de fer saccharose.

6. Procédé selon la revendication 5, **caractérisé en ce que** ce solvant est un alcool, de préférence un alcool C₍₁₋₆₎ primaire, secondaire ou tertiaire ou un alcool de benzyle ou éthylène glycol ou propylène glycol ou glycérine, de préférence méthanol, éthanol, n-propanol, isopropanol, n-butanol, butanol secondaire et/ ou butanol tertiaire, de préférence méthanol et éthanol.

7. Procédé selon la revendication 5, **caractérisé en ce que** ce solvant est un cétone, de préférence un composé de la formule C₍₁₋₃₎-alkyle-C(O)-C₍₁₋₃₎-alkyle, de préférence un composé de la formule C_{(1,2)}-alkyle-C(O)-C_{(1,2)}-alkyle, de préférence méthyle éthyle cétone ou acétone, de préférence acétone.

8. Procédé selon les revendications 6 ou 7, **caractérisé en ce qu'**un mélange de ces solvants est utilisé, de préférence un seul solvant ou un mélange de ces solvants qui comprend environ 20% en poids, 40% en poids ou 60% en poids de méthanol ou d'éthanol ou environ 20% en poids, 40% en poids ou 60% en poids d'un mélange de méthanol et éthanol.

9. Procédé selon l'une quelconque des revendications 1 à 8, **caractérisé en ce que** dans l'étape (a) le sel de fer est mélangé avec un alcali carbonate, de préférence avec 1.5 à 5.0 équivalents, de préférence avec 1.5 à 2.0 équivalents de la base de carbonate et/ ou d'un alcali hydrogénocarbonate, de préférence avec 3.0 à 10.0 équivalents, de préférence avec 3.0 à 4.0 équivalents d'une base d'hydrogénocarbonate, à chaque fois par équivalent d'ion fer, le mélange de réaction est tenu à la valeur d'acidité réglée jusqu'à ce que tout le sel de fer soit transformé en oxyhydroxyde de fer, optionnellement l'oxyhydroxyde de fer formé est prénettoyé des ions étrangers et ensuite sont ajoutés au moins 2 équivalents, de préférence 2.0 à 5.0 équivalents de sucrose, par équivalent d'ion fer.

10. Procédé selon la revendication 9, **caractérisé en ce que** l'oxyhydroxyde de fer formé est prénettoyé des ions étrangers au moyen de la décantation, de l'échange ionique, de la filtration ou de l'ultrafiltration.

11. Procédé selon l'une quelconque des revendications 1 à 10, **caractérisé en ce que** l'oxyhydroxyde de fer formé est transformé *in situ* ou à la suite d'une isolation, cependant sans nettoyage particulier des anions, directement avec du sucrose en complexe de fer saccharose.

12. Procédé selon la revendication 11, **caractérisé en ce que** l'oxyhydroxyde de fer formé est transformé *in situ* directement avec du sucrose en complexe de fer saccharose.

13. Procédé selon l'une quelconque des revendications 1 à 12, **caractérisé en ce que** dans l'étape (b) le mélange est chauffé à la température de reflux pendant au moins 0.2 heures, de préférence pendant 0.5 à 96 heures, de préférence pendant environ 1 à 8 heures, de préférence pendant environ 6 à 8 heures.

14. Procédé selon l'une quelconque des revendications 1 à 13, **caractérisé en ce que** la solution colloïdale qui comprend le complexe de fer saccharose est nettoyée d'impuretés par filtration, par ultrafiltration, par échange d'ions, par dialyse ou par une autre technique de filtration connue en soi.

15. Procédé selon l'une quelconque des revendications 1 à 14, **caractérisé en ce que** la solution aqueuse du complexe obtenu est chauffée après l'enlèvement des sels étrangers à une température de l'ordre de 70°C jusqu'à la température de reflux, optionnellement sous pression jusqu'à une température de 140°C.

16. Procédé selon l'une quelconque des revendications 1 à 15, **caractérisé en ce que** le complexe obtenue est directement précipité, filtré ou séparé par centrifugation et ensuite nettoyé.
